# EUROPEAN PATENT SPECIFICATION

(11) **EP 3 901 815 B1**
(45) Date of publication and mention of the grant of the patent: **09.09.2026**
(21) Application number: 19898062.5
(22) Date of filing: 10.09.2019
(51) Int. Cl.: G06T 19/00, A61B 6/46, G16H 40/00, G06T 12/30, G06V 20/00, G06V 10/25, G06T 15/20, A61B 6/02, G16H 30/40, A61B 6/00

(54) **IMAGE DISPLAY METHOD, APPARATUS AND DEVICE, AND COMPUTER STORAGE MEDIUM**
BILDANZEIGEVERFAHREN, GERÄT UND VORRICHTUNG SOWIE COMPUTERSPEICHERMEDIUM
PROCÉDÉ, APPAREIL ET DISPOSITIF D'AFFICHAGE D'IMAGE ET SUPPORT DE STOCKAGE INFORMATIQUE

(30) Priority: 17.12.2018 CN 201811544361
(43) Date of publication of application: 27.10.2021
(73) Proprietor: Nuctech Company Limited, TongFang Building Shuangqinglu Haidian District Beijing 100084 (CN); Tsinghua University, Beijing 100084 (CN)
(72) Inventor: ZHANG, Li, Beijing 100084 (CN); SUN, Yunda, Beijing 100084 (CN); ZHAO, Tiao, Beijing 100084 (CN); JIN, Xin, Beijing 100084 (CN); SHEN, Le, Beijing 100084 (CN); CHEN, Zhiqiang, Beijing 100084 (CN)
(74) Representative: Boult Wade Tennant LLP
(86) International application number: PCT/CN2019/105155
(87) International publication number: WO 2020/125081

(56) References cited:
- WO-A1-2018/104322
- CN-A- 1 903 139
- CN-A- 103 220 974
- CN-A- 103 460 245
- CN-A- 103 781 424
- US-A1- 2005 119 550
- US-A1- 2014 218 366
- JEROME DECLERCK * ET AL: "Automatic Registration and Alignment on a Template of Cardiac Stress and Rest Reoriented SPECT Images", IEEE TRANSACTIONS ON MEDICAL IMAGING, IEEE, USA, vol. 16, no. 6, 1 December 1997 (1997-12-01), XP011035679, ISSN: 0278-0062

## Description

### TECHNICAL FIELD

This application relates to the field of image display, and in particular to an image display method, apparatus and device and a computer storage medium.

### BACKGROUND

Images acquired by existing radiation imaging equipment are usually displayed with a carousel mode. For example, Digital Radiography (DR) images acquired by direct digital X-ray radiography systems, Computed Tomography (CT) images acquired by CT systems, and TSP images calculated by forward projection of three-dimensional reconstructed image data and so on are displayed with the carousel mode.

When images are displayed with the carousel mode, an image that is expected to be displayed may be selected by dragging a scroll bar. For example, when the images are the DR images or the TSP images, the scroll bar represents perspective-angles arranged in sequence, and a user may continuously drag the scroll bar to display the images in respective perspective-angles in succession. For another example, when the images are the CT images, the scroll bar represents tomographic positions arranged in sequence, and the user may continuously drag the scroll bar to display the images at respective tomographic positions in succession.

However, when the images are displayed by the above method, all the images are displayed by taking the size center of the images as the center of the images. Therefore, a scanned object that the user is interested in or a prohibited/suspected object area identified by a recognition algorithm will be displayed at different positions on respective images, and a certain scanned object or prohibited/suspected object area cannot be highlighted, leading to tearing of user observation.

Jerome Declerk et al "Automatic Registration and Alignmetn on a Template of Cardiac Stress and Rest Reoriented SPECT images", IEEE TRANSACTIONS ON MEDICAL IMAGING, IEEE, USA, Vol 16, no 6, 1 December 1997, ISSN: 0278-0062 discloses comparing images of a heart in a condition of stress and rest by aligning the images using the centre point of the images of the heart.

### SUMMARY

Embodiments of the application provide an image display method, apparatus and device and a computer storage medium, which can always display a target object at a target position when displaying images and thus facilitate the user to view the target object.

In an aspect, the embodiments of the application provide an image display method according to claim 1.

In another aspect, the embodiments of the application provide an image display apparatus according to claim 3.

In yet another aspect, the embodiments of the application provide an image display device, comprising: a processor; and a memory storing computer program instructions, wherein the processor, when executing the computer program instructions, implements the above-mentioned image display method.

In still another aspect, the embodiments of the application provide a computer storage medium having computer program instructions stored thereon, wherein the computer program instructions, when executed by a processor, implements the above-mentioned image display method.
The image display method, apparatus and device and the computer storage medium of the embodiments of the application can determine corresponding object positions of the target object, which the user is interested in, in the multiple first images to be displayed, process respective first images based on the object positions and the target position, and generate the second images in one-to-one correspondence with the first images and then display the second images in succession, so that the target object is always positioned at the target position when the second images are displayed. Therefore, when the user views the second images in succession, the target object, which he is interested in, can be always positioned at the same fixed position in the multiple second images, and it is unnecessary for the user to look for the target object in the images, which improves convenience of viewing the images containing the target object and the target object by the user, thereby realizing more efficient human-computer interaction, and avoiding difficulties in finding the target object due to continuous movement of the display position of the target object when there are too many objects in the images.

### BRIEF DESCRIPTION OF THE DRAWINGS

Features, advantages, and technical effects of exemplary embodiments of the application will be described below with reference to the accompany drawings.
FIG. 1 is a flow chart of an image display method provided by an embodiment of the application;
FIG. 2 is a flow chart of an image display method provided by another embodiment of the application;
FIG. 3 is a flow chart of an example of step S220 in FIG. 2;
FIG. 4 is a flow chart of an example of step S110 in FIG. 1;
FIG. 5 is a flow chart of an example of step S120 in FIG. 1;
FIGs. 6a-c are diagrams of first images in a first embodiment;
FIGs. 7a-c are diagrams of second images in the first embodiment;
FIG. 8 is a diagram of a three-dimensional image in a second embodiment;
FIG. 9 is a top projection view of the three-dimensional image shown in FIG. 8;
FIGs. 10a-c are diagrams of the first images in the second embodiment;
FIGs. 11a-c are diagrams of the second images in the second embodiment;
FIG. 12 is a structure diagram of an image display apparatus provided by an embodiment of the application;
FIG. 13 is a structure diagram of an image display apparatus provided by another embodiment of the application;
FIG. 14 is a hardware structure diagram of an image display device provided by embodiments of the application.

### DETAILED DESCRIPTION

Implementations of the application will be described in further detail below in combination with embodiments and the accompany drawings. The detailed description of the following embodiments and the accompany drawings are used to exemplarily illustrate the principles of the application instead of limiting the scopes of the application. That is, the application is not limited to the described embodiments.

In order to solve problems of the prior art, embodiments of the application provide an image display method, apparatus and device and a computer storage medium. The image display method provided by the embodiments of the application will be firstly introduced below.

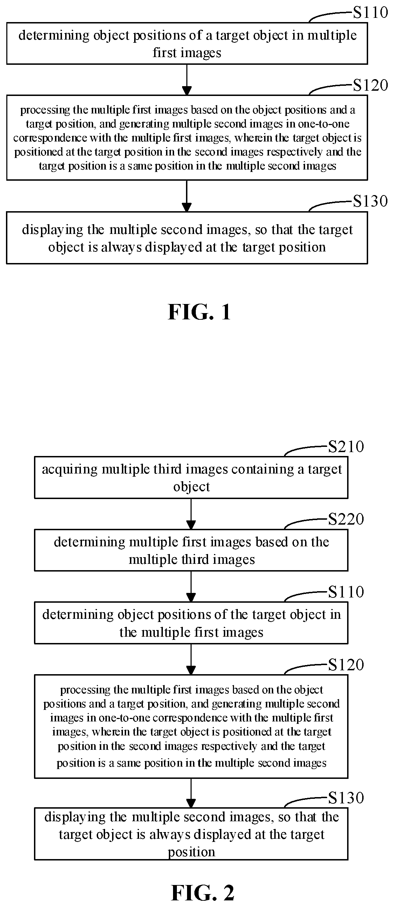

FIG. 1 shows a flow chart of an image display method provided by an embodiment of the application. As shown in FIG. 1, the image display method of the embodiment of the application includes: S110, determining object positions of a target object in multiple first images; S120, processing the multiple first images based on the object positions and a target position, and generating multiple second images in one-to-one correspondence with the multiple first images, wherein the target object is positioned at the target position in the second images respectively and the target position is a same position in the multiple second images; and S130, displaying the multiple second images.

In the embodiments of the application, it is possible to determine corresponding object positions of the target object, which the user is interested in, in the multiple first images to be displayed based on the target object, and then based on the object positions of the target object in respective first images and the target position where the target object is expected to be displayed, process respective first images, and generate the multiple second images in one-to-one correspondence with the first images, so that the target object can be always positioned at the target position when the second images are displayed in succession. Therefore, when the user views the second images in succession, the target object, which he is interested in, can be always positioned at the same fixed position in the multiple second images, and there is no need to find the target object in the images, which improves the convenience for the user to view the images and the target object.

FIG. 2 shows a flow chart of an image display method provided by another embodiment of the application. As shown in FIG. 2, before step S110 of determining object positions of a target object in multiple first images, the image display method further includes: S210, acquiring multiple third images containing the target object; and S220, determining the multiple first images based on the multiple third images.

In step S210, the multiple third images containing the target object may be acquired with radiation imaging equipment. For example, an object to be inspected containing the target object may be scanned by a DR system, and DR images of the object to be inspected acquired by scanning may be taken as the third images. For another example, the object to be inspected containing the target object may be scanned by a CT system, and CT images of the object to be inspected acquired by scanning may be taken as the third images.

The object to be inspected may be composed of multiple separate objects or may be composed of only one object.

When the object to be inspected is composed of multiple separate objects, the target object may be a focus object or a focus area selected by the user from the multiple objects, and the target object may also be a focus object or a focus area recognized by the radiation imaging equipment, wherein the focus area may be an area including a part or all of one or more objects.

When the object to be inspected is composed of only one object and the object is composed of multiple structures or contains multiple structures, the target object may be a focus structure or a focus area selected by the user from the object, and the target object may also be a focus structure or a focus area recognized by the radiation imaging equipment, where the focus area may be an area including a part or all of one or more structures.

Taking the use of the radiation imaging equipment to implement safety inspection of the object to be inspected as an example, multiple packages and/or items may be placed in the radiation imaging equipment at the same time, and these packages and/or items constitute the object to be inspected. The target object may be a package or item, which is suspected to be a prohibited or suspected object, selected by the user or recognized by the radiation imaging equipment, and the package or item is taken as the focus object. The target object may also be an area containing the package or item, which is suspected to be a prohibited or suspected object, selected by the user or recognized by the radiation imaging equipment, and the area is taken as the focus area.

Continuing to take the use of the radiation imaging equipment to implement safety inspection of the object to be inspected as an example, only one package is placed in the radiation imaging equipment, the package constitutes the object to be inspected, and the package contains multiple items therein. The target object may be an item, which is suspected to be a prohibited or suspected object, selected by the user or recognized by the radiation imaging equipment, and the item is taken as the focus object. The target object may also be an area containing the package or item, which is suspected to be a prohibited or suspected object, selected by the user or recognized by the radiation imaging equipment, and the area is taken as the focus area.

It should be noted that in practice, it may be determined whether the focus object or the focus area is taken as the target object based on practical situations.

In the embodiments of the application, the first images, the second images and the third images may be at least one of the following types of image: perspective images in respective directions containing the target object and sectional-images at respective positions containing the target object. Therefore, based on different contents corresponding to the first images, the second images and the third images, step S220 of determining the multiple first images based on the multiple third images may be classified into at least the following three cases:

### First case

When the second images and the third images are respectively the perspective images in respective directions containing the target object, step S220 of determining the multiple first images based on the multiple third images includes: taking the multiple third images as the multiple first images.

In this case, since the first images, the second images and the third images are all the perspective images in respective directions containing the target object, the third images may be directly taken as the first images for subsequent processing. For example, when the third images acquired from the radiation imaging equipment are the DR images, the DR images acquired from the radiation imaging equipment (i.e., the third images) may be directly taken as the first images to be displayed, and the first images may be processed to generate the DR images for display (i.e., the second images).

### Second case

When the second images and the third images are respectively cross-section images at respective positions containing the target object, step S220 of determining the multiple first images based on the multiple third images includes: taking the multiple third images as the multiple first images.

In this case, since the first images, the second images and the third images are allcross-section images at respective positions containing the target object, the third images may be directly taken as the first images for subsequent processing. For example, when the third images acquired from the radiation imaging equipment are the CT images, the CT images acquired from the radiation imaging equipment (i.e., the third images) may be directly taken as the first images to be displayed, and the first images may be processed to generate the CT images for display (i.e., the second images).

### Third case

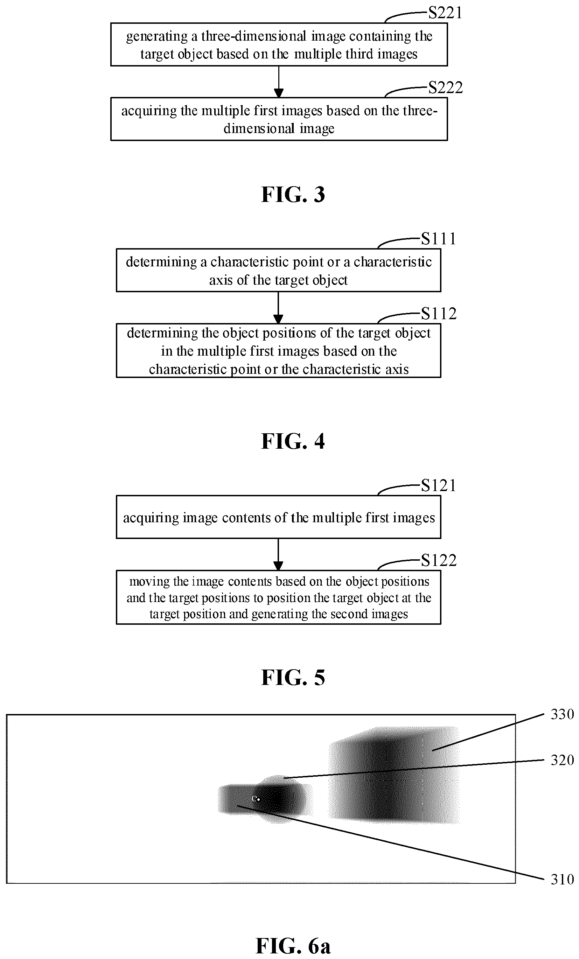

FIG. 3 shows a flow chart of an example of step S220 in FIG. 2. As shown in FIG. 3, when the third images are cross-section images at respective positions containing the target image and the second images are the perspective images in respective directions containing the target image, step S220 of determining the multiple first images based on the multiple third images includes: S221, generating a three-dimensional image containing the target object based on the multiple third images; and S222, acquiring the multiple first images based on the three-dimensional image.

In this case, since the third images are cross-section images at respective positions containing the target image and the second images are the perspective images in respective directions containing the target image, in order to implement subsequent processing, it is necessary to process the multiple third images and generate the perspective images at respective positions containing the target image as the first images. At this time, it is necessary to use the multiple third images to reconstruct the corresponding three-dimensional image thereof, acquire the perspective images of the contents in the three-dimensional image in various directions, and then take the perspective images as the first images. For example, when the third images acquired from the radiation imaging equipment are the CT images (i.e., the third images), the CT images may be used to reconstruct the three-dimensional image corresponding to the contents of the CT images, and then the three-dimensional image may be used to acquire multiple TSP images as the first images to be displayed. The first images may be processed, and then the TSP images for display (i.e., the second images) may be generated.

Therefore, the embodiments of the application can process the acquired third images based on the types of the third images and the second images to be displayed, so as to acquire the first image of the same type as the second images.

In the following, specific description of the image display method of the embodiments of the application will be continued.

FIG. 4 shows a flow chart of an example of step S110 in FIG. 1. As shown in FIG. 4, step S110 of determining object positions of a target object in multiple first images may include: S111, determining a characteristic point or a characteristic axis of the target object; and S112, determining the object positions of the target object in the multiple first images based on the characteristic point or the characteristic axis.

In step S111, the characteristic point may be the center point of the target object or a point on any surface or any side of the target object, and the characteristic axis may be an axis passing through the target object. Specifically, the axis should be parallel to a rotation axis of the object to be inspected when multiple perspective images containing the target object are acquired.

In step S112, when the characteristic point or the characteristic axis is determined, the position of the characteristic point or the characteristic axis in the first image may be taken as the object position of the target object in the first image.

Specifically, when the position of the characteristic point is used as the object position of the target object, the position of the characteristic point includes not only its horizontal and vertical coordinates in the image but also a depth coordinate of the characteristic point (that is, the distance of the characteristic point from the plane of the image in the direction perpendicular to the plane of the image). That is, the position of the characteristic point is three-dimensional coordinates of the characteristic point in the first image. When the position of the characteristic axis is used as the object position of the target object, the position of the characteristic axis includes not only its coordinates in the plane of the image but also a depth coordinate of the characteristic axis (that is, the distance of the characteristic axis from the plane of the image in the direction perpendicular to the plane of the image). That is, the position of the characteristic axis is three-dimensional coordinates of the characteristic axis in the first image.

In the embodiments of the application, the target position is the same position in the multiple second images, and the target position may be a display position determined based on the object positions, or a preset display position other than the object positions.

When the target position is the display position determined based on the object positions, the display position may be the position of a certain point or axis selected from the object positions. Specifically, the user may select any one of the first images, and take the object position of the characteristic point or the characteristic axis of the target object in the first image as the target position.

When the target position is the preset display position other than the object positions, the display position may be a preset position of a certain point or axis, and may also be a preset display area. When the display position is the preset display area, the position of any point or axis in the display area may be randomly selected as the target position. But it should be noted that, when the position is randomly selected for the first time, the target position is fixed to the position.

It should be noted that the preset display position other than the object positions may also be a default display position, that is, the size center (center point or center line) of the second image.

Therefore, in the embodiments of the application, the characteristic point or the characteristic axis may be used to represent the target object, so that the representation of the target object is simple, and the object position representing the characteristic point or the characteristic axis of the target object may be easily acquired.

FIG. 5 shows a flow chart of step S120 in FIG. 1. As shown in FIG. 5, step S120 of processing the multiple first images based on the object positions and a target position and generating multiple second images in one-to-one correspondence with the multiple first images may include: S121, acquiring image contents of the multiple first images; and S122, based on the object positions and the target position, moving the image contents to position the target object at the target position and generating the second images.

That is, in the embodiments of the application, the image content of the first image may be moved as a whole to position the characteristic point or the characteristic axis of the target object at the target position. Therefore, in the embodiments of the application, the target object may be positioned at the target position without changing the size, layout and so on of the image content so as to generate the second image.

Based on the above processing, when step S130 of displaying the second images in succession of the embodiments of the application is implemented, the target object may be fixedly displayed at the target position, so that the user can view the multiple second images and the target object conveniently. The mode of displaying in succession may be: when two-dimensional images are displayed, the characteristic point or the characteristic axis of the target image is fixedly positioned at the target position, and respective DR images or CT images are displayed in succession; when three-dimensional images are displayed, the characteristic axis of the target image is fixedly positioned at the target position, and respective TSP images are displayed in succession while the object to be inspected is rotated with the characteristic axis as the rotation axis.

It should be noted that, in the embodiments of the application, when the second images are zoomed in or out, the characteristic point or the characteristic axis of the target object is fixedly displayed at the target position without change. Therefore, it can be further convenient for the user to view the multiple second images and the target image of the object to be inspected.

For example, when the multiple first images are respectively the two-dimensional images, the target object and the characteristic point or the characteristic axis of the target object may be firstly determined on respective first images (the same target object has only the same point or axis as its characteristic point or characteristic axis), and then specific positions (i.e., the object positions) of the characteristic point or the characteristic axis of the target object in respective first images may be determined, the specific positions being the three-dimensional coordinates of the characteristic point or the characteristic axis. Then, the object position corresponding to one of the first images may be selected as the target position from the multiple object positions corresponding to the multiple first images. Finally, the image contents of the other first images are moved as a whole to make the object positions corresponding to the first images exactly the same as the three-dimensional coordinates of the target position and thus generate new images as the second images, so that the highlight display of the target object is realized when the second images are displayed in succession.

For another example, when the multiple first images are respectively images of different perspectives of the three-dimensional image, the target object and the characteristic axis of the target object may be firstly determined on respective first images (the same target object has only the same axis as its characteristic axis), and then specific positions (i.e., the object positions) of the feature axis of the target object in respective first images may be determined, the specific positions being the three-dimensional coordinates of the characteristic axis. Then, the object position corresponding to one of the multiple first images may be selected as the target position from the multiple object positions corresponding to the multiple first images. Finally, the image contents of the other first images are moved as a whole to make the object positions corresponding to the first images exactly the same as the three-dimensional coordinates of the target position and thus generate new images as the second images, so that the highlight display of the target object is realized when the second images corresponding to respective perspectives of the three-dimensional image are displayed by rotating the three-dimensional image with the characteristic axis as the rotation axis.

In the embodiments of the application, the DR images, the CT images and the TSP images may be the second images as the two-dimensional images displayed with the carousel mode, and the TSP images may also be the second images displayed with respective perspectives by rotating the three-dimensional image around the rotation axis.

In the following, the display method of the embodiments of the application is described in combination with two specific embodiments.

### First embodiment

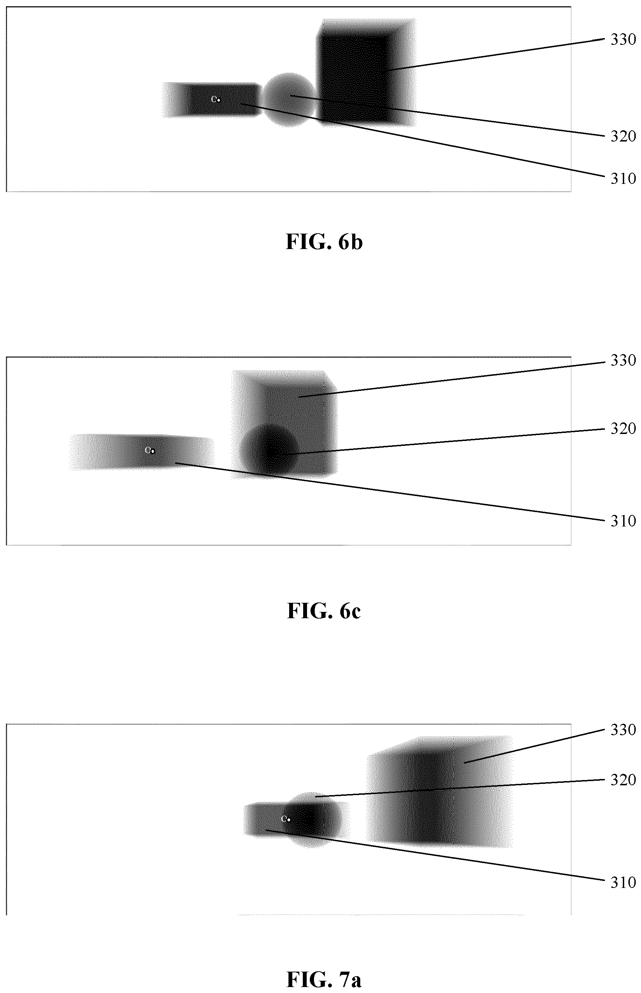

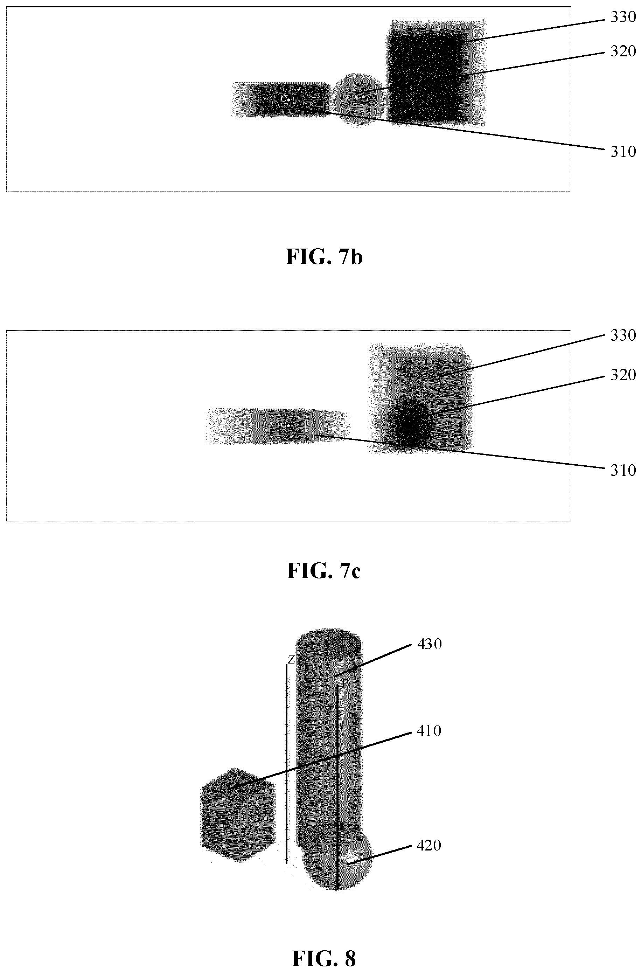

FIGs. 6a, 6b and 6c respectively show diagrams of the first images in a first embodiment. FIGs. 7a, 7b and 7c respectively show diagrams of the second images in the first embodiment.

As shown in FIGs. 6a-c, three first images respectively represent three perspective images acquired by imaging the object to be inspected from three directions. The image contents of the first images include a cuboid 310, a sphere 320, and a cube 330. If it is expected to highlight the cuboid 310, since its positions on the first images at respective angles are different, it is difficult for the user's eyes to locate the position of the cuboid 310 immediately when the perspective images are displayed with the carousel mode.

Therefore, the cuboid 310 may be specified as the target object by the user firstly. For example, the cuboid 310 may be selected as the target object by the user on the first image shown in FIG. 6b, and the center point O of the cuboid 310 may be taken as the characteristic point (the characteristic point may be specified by the customer or the system). Secondly, the three-dimensional coordinates of the center point O may be determined as the target position, wherein the depth coordinate in the three-dimensional coordinates may be set by the user or determined by the system based on a predetermined algorithm. Thirdly, the system may determine the three-dimensional coordinates of the center point O of the cuboid 310 in the first images shown in FIGs. 6a and 6c as the object positions. Finally, the image contents of the first images shown in FIGs. 6a and 6c are moved to position the center point O at the position of the center point O in FIG.6b (i.e., the target position), and then the second images in FIGs. 7a-7c are generated and displayed with the carousel mode while the highlight display of the cuboid 310 is realized.

### Second embodiment

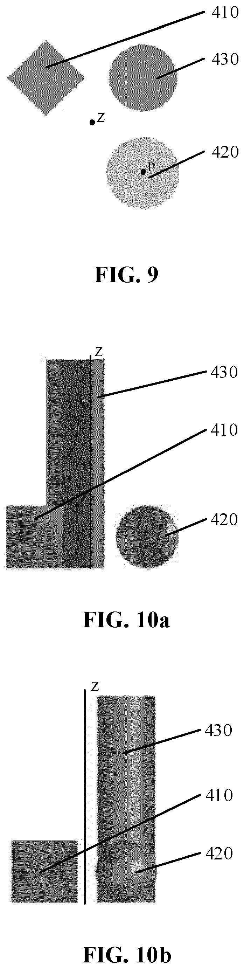

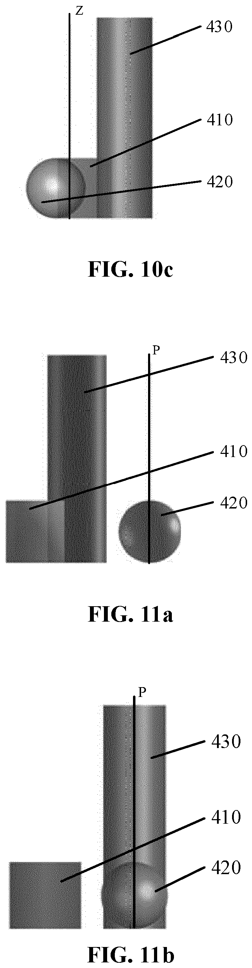

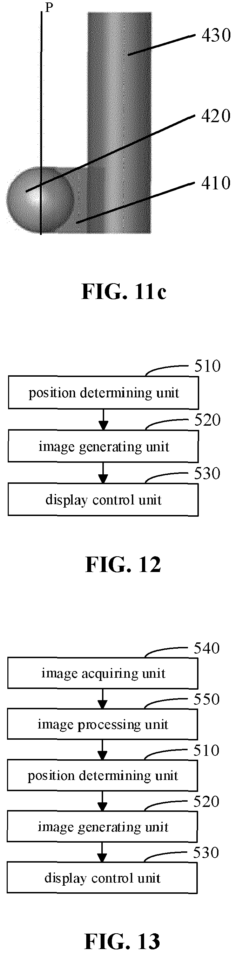

FIG. 8 shows a diagram of a three-dimensional image in a second embodiment. FIG. 9 is a top projection view of the three-dimensional image shown in FIG. 8. FIGs.10a, 10b and 10c respectively show diagrams of the first images in the second embodiment. FIGs. 11a, 11b and 11c respectively show diagrams of the second images in the second embodiment.

As shown in FIG. 8, the three-dimensional image of the object to be inspected is displayed, and its corresponding top projection view is shown in FIG. 9. As shown in FIG. 10a-c, three first images respectively represent three perspective images acquired by imaging the object to be inspected from three directions of 120°, 180°, and 240°. The image contents of the first images include a cuboid 410, a sphere 420, and a cylinder 430.

As shown in FIGs. 10a-c, when the perspective images are acquired with Z axis, the sphere 420 is positioned at different positions on the perspective images of different angles. Therefore, when the three-dimensional image is rotated with Z axis as the rotation axis, the sphere 420 will be displayed at different positions. The sphere 420 cannot be highlighted, which makes it difficult for the user's eyes to locate the position of the sphere 420 immediately.

Therefore, the sphere 420 may be firstly specified as the target object by the user. For example, the sphere 420 may be selected as the target object by the user on the first image shown in FIG. 10a, and the axis 421, which is parallel to Z axis, of the sphere 420 may be taken as the characteristic axis (the characteristic point may be specified by the customer or the system). Secondly, the three-dimensional coordinates of the axis 421 may be determined as the target position, and the depth coordinate in the three-dimensional coordinates may be set by the user or determined by the system based on a predetermined algorithm. Thirdly, the system may determine the three-dimensional coordinates of the axis (i.e., P axis in the figure) of the sphere 420 in the first images shown in FIGS. 10b and 10c as the object positions. Finally, the image contents of the first images shown in FIG. 10b and FIG. 10c are moved to position P axis at the position of P axis in FIG. 10a (i.e., the target position) and then the second images shown in FIG. 11a-11c are generated. The second images shown in FIGs. 11a-11c are displayed by rotating the three-dimensional image with P axis as the rotation axis, so that the highlight display of the sphere 420 is realized and the sphere 420 remains stationary.

In conclusion, the image display method of the embodiments of the application may firstly determine corresponding object positions of the target object, which the user is interested in, in the multiple first images to be displayed based on the target object, and then process respective first images based on the object positions of the target object in respective first images and the target position where the target object is expected to be displayed, and generate the multiple second images in one-to-one correspondence with respective first image, so that the target object is always positioned at the target position when the second images are displayed in succession. Therefore, when the user views the images in succession, he can see the second images containing the target object that is fixedly displayed at the same target position without looking for the target object in the images, which improves the convenience for the user to view the images and the target object. At the same time, when the embodiments of the application are applied to safety inspection using the radiation imaging equipment, since the area that the user is interested in or the dangerous object area given by the recognition algorithm can be highlighted, the efficiency of the safety inspection can also be improved.

FIG. 12 shows a structure diagram of an image display apparatus provided by an embodiment of the application. As shown in FIG. 12, the image display apparatus provided by the embodiment of the application includes: a position determining unit 510 configured to determine object positions of a target object in multiple first images; an image generating unit 520 configured to process the multiple first images based on the object positions and a target position and generate multiple second images in one-to-one correspondence with the multiple first images, wherein the target object is positioned at the target position in the second images respectively, and the target position is a same position in the multiple second images; and a display control unit 530 configured to display the multiple second images.

In the embodiments of the application, the position determining unit 510 may determine corresponding object positions of the target object, which the user is interested in, in the multiple first images to be displayed based on the target object, and the image generating unit 520 may process respective first images based on the object positions of the target object in respective first images and the target position where the target object is expected to be displayed and generate the multiple second images in one-to-one correspondence with respective first images, so that the display control unit 530 may always position the target object at the target position when displaying the second images in succession. Therefore, when the user views the images in succession, he can view the second images containing the target object that is fixedly displayed at the same target position without looking for the target object in the images, which improves the convenience for the user to view the images and the target object.

FIG. 13 shows a structure diagram of an image display apparatus provided by another embodiment of the application. As shown in FIG. 13, the image display device further includes: an image acquiring unit 540 configured to acquire multiple third images containing the target object; and an image processing unit 550 configured to determine the multiple first images based on the multiple third images.

In the embodiments of the application, the image acquiring unit 540 may acquire the third images of the object to be inspected from an image acquisition device (for example, the radiation imaging equipment), and the image processing unit 550 may process the acquired third images based on the types of the third images and the second images to be displayed, so as to obtain the first images of the same type as the second images.

Specifically, when the second images and the third images are respectively perspective images in respective directions containing the target object or the second images and the third images are respectively cross-sectional images at respective positions containing the target object, the image processing unit 550 is further configured to take the multiple third images as the multiple first images. When the third images are cross-section images at respective positions containing the target image and the second images are the perspective images in respective directions containing the target image, the image processing unit 550 is further configured to: generate a three-dimensional image containing the target object based on the multiple third images; and acquire the multiple first images based on the three-dimensional image.

In the embodiments of the application, the position determining unit 510 is further configured to: determine a characteristic point or a characteristic axis of the target object; and determine the object positions of the target object in the multiple first images based on the characteristic point or characteristic axis.

Therefore, in the embodiments of the application, the position determining unit 510 may use the characteristic point or the characteristic axis to represent the target object, so that the representation of the target object is simple and the object positions of the characteristic point or the characteristic axis representing the target object may be easily acquired.

In the embodiments of the application, the image generating unit 520 is further configured to: acquire image contents of the multiple first images; and move the image contents based on the object positions and the target position to position the target object at the target position, and generate the second images.

That is, in the embodiments of the application, the image generating unit 520 may move the image content of the first image as a whole to position the characteristic point or the characteristic axis at the target position. Therefore, in the embodiments of the application, the target object may be positioned at the target position without changing the size, layout and so on of the image content, so as to generate the second image.

In the embodiments of the application, when the display control unit 530 displays the second images in succession, it may fixedly display the target object at the target position, thereby facilitating the user to view the multiple second images and the target object. The mode of displaying in succession may be: when two-dimensional images are displayed, the characteristic point or the characteristic axis of the target image is fixedly positioned at the target position, and respective DR images or CT images are displayed in succession; when three-dimensional images are displayed, the characteristic axis of the target image is fixedly positioned at the target position, and respective TSP images are displayed in succession while the object to be inspected is rotated with the characteristic axis as the rotation axis.

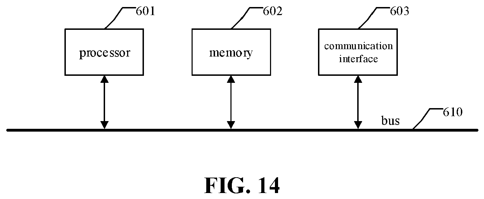

FIG. 14 shows a hardware structure diagram of an image display device provided by embodiments of the present application.

The image display device may include a processor 601 and a memory 602 storing computer program instructions thereon.

Specifically, the processor 601 may include a Central Processing Unit (CPU), a specific integrated circuit (Application Specific Integrated Circuit, ASIC), or one or more integrated circuits configured to implement the embodiments of the application.

The memory 602 may include a mass storage for data or instructions. For example rather limitation, the memory 602 may include a Hard Disk Drive (HDD), a floppy disk drive, a flash memory, an optical disk, a magneto-optical disk, a magnetic tape, or a Universal Serial Bus (USB) drive, or a combination of two or more of them. Where appropriate, the memory 602 may include a removable or non-removable (or fixed) medium. Where appropriate, the memory 602 may be inside or outside of an integrated gateway disaster recovery device. In a specific embodiment, the memory 602 is a non-volatile solid-state memory. In a specific embodiment, the memory 602 includes a read-only memory (ROM). Where appropriate, the ROM may be a mask-programmed ROM, a programmable ROM (PROM), an erasable PROM (EPROM), an electrically erasable PROM (EEPROM), an electrically rewritable ROM (EAROM) or a flash memory or a combination of two or more of them.

The processor 601 reads and executes the computer program instructions stored in the memory 602 to implement any one of the image display methods in the foregoing embodiments.

In an example, the image display device may further include a communication interface 603 and a bus 610. As shown in FIG. 14, the processor 601, the memory 602 and the communication interface 603 are connected to one another via the bus 610 to implement mutual communication.

The communication interface 603 is mainly used to implement communications between various modules, apparatuses, units and/or devices in the embodiments of the application.

The bus 610 includes hardware, software, or both of them, and couples components of an online data traffic accounting device to each other. By way of example rather than limitation, the bus may include an Accelerated Graphics Port (AGP) or another graphics bus, an Enhanced Industry Standard Architecture (EISA) bus, a Front Side Bus (FSB), a Hypertransport (HT) interconnect, an Industry Standard Architecture (ISA) Bus, an Infinite Bandwidth Interconnect, a Low Pin Count (LPC) Bus, a Memory Bus, a Micro Channel Architecture (MCA) Bus, a Peripheral Component Interconnect (PCI) Bus, a PCI-Express (PCI-X) Bus, a Serial Advanced Technology Attachment (SATA) bus, a Video Electronics Standards Association Local (VLB) bus or other suitable buses or a combination of two or more of the abovementioned buses. Where appropriate, the bus X10 may include one or more buses. Although a specific bus is described and shown in the embodiments of the application, the application encompasses any suitable buses or interconnections.

The image display device may firstly determine corresponding object positions of the target object, which the user is interested in, in the multiple first images to be displayed based on the target object, and then process respective first images based on the object positions of the target object in respective first images and the target position where the target object is expected to be displayed and generate the multiple second images in one-to-one correspondence with respective first images, so that when the second images are displayed in succession, the target object is always positioned at the target position, thereby realizing the image display method and apparatus described in conjunction with the above drawings.

In addition, embodiments of the application provide a computer storage medium to implement the image display method described in combination with the above embodiments. The computer storage medium stores computer program instructions, which when executed by a processor, implement any of the image display methods in the foregoing embodiments.

It should be clear that the application is not limited to the specific configuration and processing described above and shown in the drawings. For purpose of brevity, detailed descriptions of known methods are omitted herein. In the above embodiments, several specific steps are described and shown as examples. However, the process of the method of the application is not limited to the specific steps described and shown herein. After understanding the spirits of the application, those skilled in the art can make various changes, modifications and additions, or change the order of the steps.

The function blocks shown in the above described structural block diagrams may be implemented in hardware, software, firmware, or a combination thereof. When implemented in hardware, the function blocks may be, for example, electronic circuits, application specific integrated circuits (ASICs), appropriate firmware, plug-ins, function cards, and so on. When implemented in software, the elements of the application are programs or code segments for implementing required tasks. The programs or code segments may be stored in the machine-readable medium, or transmitted by data signals carried in carriers on a transmission medium or a communication link. "Machine-readable medium" may include any medium that can store or transfer information. Examples of the machine-readable medium include electronic circuits, semiconductor memory devices, ROMs, flash memories, erasable ROMs (EROMs), floppy disks, CD-ROMs, optical disks, hard disks, fiber optic media, radio frequency (RF) links, and so on. The code segments may be downloaded via a computer network such as Internet, an intranet, etc.

It should also be noted that, in the exemplary embodiments mentioned in the application, some methods or systems are described based on a series of steps or apparatuses. However, the application is not limited to the order of the above steps. That is, the steps may be performed in the order described in the embodiments or in a different order from that in the embodiments, or several steps may be executed simultaneously.

Although the application has been described with reference to preferred embodiments, various modifications may be made to the application and components of the application may be replaced with equivalents without departing from the scopes of the application. In particular, as long as there is no structural conflict, various technical features mentioned in the embodiments may be combined in any manner. The application is not limited to specific embodiments disclosed herein, and instead, it includes all technical solutions that fall within the scopes of the claims.

## Claims

1. An image display method, comprising:
acquiring multiple third images containing a target object (S210), wherein the third images are cross-sectional images at respective positions containing the target object;
generating a three-dimensional image containing the target object based on the multiple third images (S221);
acquiring multiple first images based on the three-dimensional image (S222);
determining a characteristic point or a characteristic axis of the target object (S111);
determining object positions of the target object in the multiple first images based on the characteristic point or the characteristic axis (S112);
acquiring image contents of the multiple first images (S121);
moving the image contents of the multiple first images based on the object positions and a target position to position the characteristic point or the characteristic axis of the target object at the target position and generating second images (S122), wherein the target object is positioned at the target position in the second images respectively, the target position is the same position in the multiple second images, and the second images are perspective images in respective directions containing the target image; and
displaying the multiple second images (S130).

2. The image display method according to claim 1, wherein the target position is:
a display position determined based on the object positions; or
a preset display position other than the object positions.

3. An image display apparatus, comprising:
an image acquiring unit (540) configured to acquire multiple third images containing a target object, wherein the third images are cross-sectional images at respective positions containing the target object;
an image processing unit (550) configured to generate a three-dimensional image containing the target object based on the multiple third images, and acquire multiple first images based on the three-dimensional image;
a position determining unit (510) configured to determine a characteristic point or a characteristic axis of the target object, and determine object positions of the target object in the multiple first images based on the characteristic point or the characteristic axis;
an image generating unit (520) configured to acquire image contents of the multiple first images, move the image contents of the multiple first images based on the object positions and a target position to position the characteristic point or the characteristic axis of the target object at the target position and generate second images, wherein the target object is positioned at the target position in the second images respectively, the target position is the same position in the multiple second images, and the second images are perspective images in respective directions containing the target image; and
a display control unit (530) configured to display the multiple second images.

4. An image display device, comprising:
a processor (601); and
a memory (602) storing computer program instructions, wherein
the processor, when executing the computer program instructions, implements the image display method according to any one of claims 1-2.

5. A computer storage medium having computer program instructions stored thereon, wherein the computer program instructions, when executed by a processor, implement the image display method according to any one of claims 1-2.

## Patentansprüche

1. Bildanzeigeverfahren, umfassend:
Erfassen mehrerer dritter Bilder, die ein Zielobjekt enthalten (S210), wobei die dritten Bilder Querschnittsbilder an jeweiligen Positionen sind, die das Zielobjekt enthalten;
Erzeugen eines dreidimensionalen Bildes, das das Zielobjekt enthält, basierend auf den mehreren dritten Bildern (S221);
Erfassen mehrerer erster Bilder basierend auf dem dreidimensionalen Bild (S222);
Bestimmen eines charakteristischen Punktes oder einer charakteristischen Achse des Zielobjekts (S111);
Bestimmen von Objektpositionen des Zielobjekts in den mehreren ersten Bildern basierend auf dem charakteristischen Punkt oder der charakteristischen Achse (S112);
Erfassen von Bildinhalten der mehreren ersten Bilder (S121);
Verschieben der Bildinhalte der mehreren ersten Bilder basierend auf den Objektpositionen und einer Zielposition, um den charakteristischen Punkt oder die charakteristische Achse des Zielobjekts an der Zielposition zu positionieren, und Erzeugen von zweiten Bildern (S122), wobei das Zielobjekt in den zweiten Bildern jeweils an der Zielposition positioniert ist, die Zielposition in den mehreren zweiten Bildern dieselbe Position ist und die zweiten Bilder perspektivische Bilder in jeweiligen Richtungen sind, die das Zielbild enthalten; und
Anzeigen der mehreren zweiten Bilder (S130).

2. Bildanzeigeverfahren nach Anspruch 1, wobei die Zielposition:
eine basierend auf den Objektpositionen bestimmte Anzeigeposition ist; oder
eine voreingestellte Anzeigeposition mit Ausnahme von den Objektpositionen.

3. Bildanzeigegerät, umfassend:
eine Bilderfassungseinheit (540), die konfiguriert ist, mehrere dritte Bilder zu erfassen, die ein Zielobjekt enthalten, wobei die dritten Bilder Querschnittsbilder an jeweiligen Positionen sind, die das Zielobjekt enthalten;
eine Bildverarbeitungseinheit (550), die konfiguriert ist, basierend auf den mehreren dritten Bildern ein dreidimensionales Bild zu erzeugen, das das Zielobjekt enthält, und basierend auf dem dreidimensionalen Bild mehrere erste Bilder zu erfassen;
eine Positionsbestimmungseinheit (510), die konfiguriert ist, einen charakteristischen Punkt oder eine charakteristische Achse des Zielobjekts zu bestimmen und basierend auf dem charakteristischen Punkt oder der charakteristischen Achse Objektpositionen des Zielobjekts in den mehreren ersten Bildern zu bestimmen;
eine Bilderzeugungseinheit (520), die konfiguriert ist, Bildinhalte der mehreren ersten Bilder zu erfassen, die Bildinhalte der mehreren ersten Bilder basierend auf den Objektpositionen und einer Zielposition zu verschieben, um den charakteristischen Punkt oder die charakteristische Achse des Zielobjekts an der Zielposition zu positionieren, und zweite Bilder zu erzeugen, wobei das Zielobjekt in den zweiten Bildern jeweils an der Zielposition positioniert ist, wobei die Zielposition in den mehreren zweiten Bildern dieselbe Position ist und die zweiten Bilder perspektivische Bilder in jeweiligen Richtungen sind, die das Zielbild enthalten; und
eine Anzeigesteuereinheit (530), die konfiguriert ist, die mehreren zweiten Bilder anzuzeigen.

4. Bildanzeigevorrichtung, umfassend:
einen Prozessor (601); und
einen Speicher (602), der Computerprogrammbefehle speichert, wobei
der Prozessor bei der Ausführung der Computerprogrammbefehle das Bildanzeigeverfahren nach einem der Ansprüche 1 bis 2 durchführt.

5. Computerspeichermedium, auf dem Computerprogrammbefehle gespeichert sind, wobei die Computerprogrammbefehle bei Ausführung durch einen Prozessor das Bildanzeigeverfahren nach einem der Ansprüche 1 bis 2 durchführen.

## Revendications

1. Procédé d'affichage d'image, comportant :
l'acquisition de multiples troisièmes images contenant un objet cible (S210), dans lequel les troisièmes images sont des images en coupe transversale à des positions respectives contenant l'objet cible ;
la génération d'une image tridimensionnelle contenant l'objet cible sur la base des multiples troisièmes images (S221) ;
l'acquisition de multiples premières images sur la base de l'image tridimensionnelle (S222) ;
la détermination d'un point caractéristique ou d'un axe caractéristique de l'objet cible (S111) ;
la détermination de positions d'objet de l'objet cible dans les multiples premières images sur la base du point caractéristique ou de l'axe caractéristique (S112) ;
l'acquisition de contenus d'images des multiples premières images (S121) ;
le déplacement des contenus d'images des multiples premières images sur la base des positions d'objet et d'une position cible afin de positionner le point caractéristique ou l'axe caractéristique de l'objet cible à la position cible et la génération de deuxièmes images (S122), l'objet cible étant positionné à la position cible dans les deuxièmes images respectivement, la position cible étant la même position dans les multiples deuxièmes images, et les deuxièmes images étant des images en perspective dans des directions respectives contenant l'image cible ; et
l'affichage des multiples deuxièmes images (S130).

2. Procédé d'affichage d'image selon la revendication 1, dans lequel la position cible est :
une position d'affichage déterminée sur la base des positions d'objet ; ou
une position d'affichage prédéfinie autre que les positions d'objet.

3. Appareil d'affichage d'image, comportant :
une unité d'acquisition d'images (540) configurée pour acquérir de multiples troisièmes images contenant un objet cible, dans lequel les troisièmes images sont des images en coupe transversale à des positions respectives contenant l'objet cible ;
une unité de traitement d'images (550) configurée pour générer une image tridimensionnelle contenant l'objet cible sur la base des multiples troisièmes images, et acquérir de multiples premières images sur la base de l'image tridimensionnelle ;
une unité de détermination de position (510) configurée pour déterminer un point caractéristique ou un axe caractéristique de l'objet cible, et déterminer les positions d'objet de l'objet cible dans les multiples premières images sur la base du point caractéristique ou de l'axe caractéristique ;
une unité de génération d'images (520) configurée pour acquérir les contenus d'images des multiples premières images, déplacer les contenus d'images des multiples premières images sur la base des positions d'objet et d'une position cible afin de positionner le point caractéristique ou l'axe caractéristique de l'objet cible à la position cible et générer de deuxièmes images, l'objet cible étant positionné à la position cible dans les deuxièmes images respectivement, la position cible étant la même position dans les multiples deuxièmes images, et les deuxièmes images étant des images en perspective dans des directions respectives contenant l'image cible ; et
une unité de commande d'affichage (530) configurée pour afficher les multiples deuxièmes images.

4. Dispositif d'affichage d'image, comportant :
un processeur (601) ; et
une mémoire (602) stockant des instructions de programme informatique, dans lequel
le processeur, lors de l'exécution des instructions de programme informatique, met en œuvre le procédé d'affichage d'image selon l'une quelconque des revendications 1 et 2.

5. Support de stockage informatique ayant des instructions de programme informatique stockées, dans lequel les instructions de programme informatique, lorsqu'elles sont exécutées par un processeur, mettent en œuvre le procédé d'affichage d'image selon l'une quelconque des revendications 1 et 2.
